# EUROPÄISCHE PATENTSCHRIFT

(11) **EP 2 168 631 B1**
(45) Veröffentlichungstag und Bekanntmachung des Hinweises auf die Patenterteilung: **05.08.2015**
(21) Anmeldenummer: 09168729.3
(22) Anmeldetag: 26.08.2009
(51) Int. Cl.: A61N 1/372, A61N 1/362

(54) **Elektrisches Therapiesystem und Therapiegerät**
Electric therapy system and therapy device
Système électrique de thérapie et appareil de thérapie

(30) Priorität: 25.09.2008 DE 102008042355
(43) Veröffentlichungstag der Anmeldung: 31.03.2010
(73) Patentinhaber: Biotronik CRM Patent AG, 6341 Baar (CH)
(72) Erfinder: Hennig, Carsten, 13591, Berlin (DE); Elsner, Joachim, 14059, Berlin (DE); Gromotka, Bernhard, 12359, Berlin (DE)
(74) Vertreter: Galander, Marcus

(56) Entgegenhaltungen:
- WO-A1-02/24064
- US-A1- 2005 203 582
- US-A1- 2006 030 904

## Beschreibung

Die Erfindung betrifft ein elektrisches Therapiesystem mit einem Therapiegerät, beispielsweise einem Implantat, und einem externen Gerät, beispielsweise einem Programmiergerät. Das Therapiegerät und das externe Gerät weisen jeweils eine Datenkommunikationsschnittstelle auf, die zum Durchführen einer bidirektionalen drahtlosen Datenübertragung zum jeweils anderen Gerät ausgebildet ist. Die Datenkommunikationsschnittstellen beinhalten jeweils eine Datenkommunikationssteuereinheit oder sind mit einer solchen verbunden.

Derartige elektrische Therapiesysteme sind grundsätzlich bekannt. Das Therapiegerät eines solchen Systems kann beispielsweise ein Implantat, wie ein implantierbarer Herzschrittmacher, Cardioverter/Defibrillator oder dergleichen, sein. Das externe Gerät kann ein Programmiergerät für ein derartiges Therapiegerät sein oder ein sog. Patientengerät, das als Relaisstation zwischen Implantat und einem zentralen Servicecenter dient.

Die Datenkommunikationsschnittstelle des Therapiegeräts (Therapiegerät-Datenkommunikationsschnittstelle) und die Datenkommunikationsschnittstelle des externen Geräts (externe Datenkommunikationsschnittstelle) weisen dabei jeweils einen Sender und einen Empfänger auf (zusammen auch als Transceiver bezeichnet), um eine bidirektionale Datenkommunikation zu erlauben.

Im Falle von implantierbaren Herzschrittmachern oder Cardiovertern/Defibrillatoren besitzt das Implantat, also das Therapiegerät, häufig auch zwei Datenkommunikationsschnittstellen. Häufig verwendet eine dieser Therapiegerät-Datenkommunikationsschnittstellen für die drahtlose Datenkommunikation ein magnetisches Wechselfeld. Diese Datenkommunikation wird üblicherweise zwischen einem Programmiergerät und dem Implantat verwendet und hat nur eine sehr kurze Reichweite. Diese Datenkommunikation wird angewandt, wenn sich ein Patient mit einem Herzschrittmacher bei seinem betreuenden Arzt befindet. Der Arzt kann dann einen entsprechenden Programmierkopf des Programmiergerätes auf den Körper des Patienten auflegen und so die kurzreichweitige Datenkommunikation zwischen Implantat und Programmierkopf über ein magnetisches Wechselfeld ermöglichen. Eine solche Datenkommunikationsschnittstelle ist in WO 02/24064A offenbart.

Ein Therapiesystem nach dem einleitenden Teil von Anspruch 1 ist aus US2005203582 bekannt.

Eine zweite Therapiegerät-Datenkommunikationsschnittstelle ist häufig für eine etwas länger reichweitige Datenkommunikation mit einem externen Gerät vorgesehen. Das externe Gerät kann entweder ein Programmiergerät sein, oder ein Patientengerät, welches als Relaisstation für eine Datenkommunikation zwischen einem Implantat und einem entfernten Servicecenter dient. Hierbei erfolgt die Datenkommunikation möglicherweise über ein elektrisches Wechselfeld im sog. MICS-Band. Das MICS-Band ist ein spezielles Frequenzband für die Datenkommunikation zwischen Implantaten und externen Geräten.

In jedem Fall muss die wenigstens eine Therapiegerät-Datenkommunikationsschnittstelle des Implantats von einer implantatsinternen Energiequelle versorgt werden, die typischerweise eine Batterie ist, welche notwendigerweise nur eine begrenzte Kapazität hat. Ist die Batterie eines Implantats, beispielsweise eines implantierbaren Herzschrittmachers, erst einmal erschöpft, muss der Herzschrittmacher im Rahmen einer Operation explantiert und durch einen neuen ersetzt werden. Daher besteht der Bedarf, den Energieverbrauch des Therapiegerätes so gering wie möglich zu halten. Diese Anforderung wird auch an den Energieverbrauch für die bidirektionale Datenverbindung gestellt. Aufgabe der Erfindung, welche in Anspruch 1 definiert ist, ist es somit, ein Therapiesystem und ein Therapiegerät anzugeben, die eine energiesparende und sichere Datenkommunikation erlaubt.

Erfindungsgemäß wird diese Aufgabe durch ein elektrisches Therapiesystem gelöst, bei dem wenigstens das Therapiegerät dazu ausgebildet ist, eine Datenübertragung in wenigstens zwei unterschiedlichen Datenübertragungsmodi zu erlauben und wobei die Auswahl eines Datenübertragungsmodus durch eine Datenkommunikationssteuereinheit anhand wenigstens eines vorgegebenen Auswahlkriteriums erfolgt.

Die Datenübertragungsmodi unterscheiden sich dabei vorzugsweise wenigstens hinsichtlich einer Paketkonfiguration der übertragenen Datenpakete und/oder eines Protokolls über die Datenübertragung. Dabei impliziert die Verwendung unterschiedlicher Protokolle für eine paketorientierte Datenübertragung in der Regel auch unterschiedliche Paketkonfigurationen.

Erfindungsgemäß erfolgt in jedem Fall die Umschaltung zwischen verschiedenen Datenübertragungsmodi durch die Datenkommunikationssteuereinheit automatisch gesteuert und vorzugsweise nahtlos, so dass es beim Umschalten nicht jeweils zu einem Abbruch einer bestehenden Datenkommunikationsverbindung und zum Aufbau einer neuen Datenkommunikationsverbindung kommt, sondern dass eine bereits bestehende Datenkommunikationsverbindung in einem jeweils anderen Datenübertragungsmodus fortgeführt wird. Auf diese Weise wird vermieden, dass jeweils ein neuer Verbindungsaufbau erforderlich ist, bei dem die Verwendungsparameter zwischen Therapiegerät und externem Gerät einschließlich einer Authentifizierungsinformation ausgetauscht werden müssen. Ein solcher Prozess nähme Zeit in Anspruch und kann gar manuelle Aktionen eines Anwenders erfordern.

Vorzugsweise ist die Datenkommunikationssteuereinheit dazu ausgebildet, einen jeweiligen der möglichen Datenkommunikationsmodi anhand wenigstens eines der folgenden Auswahlkriterien durchzuführen:
- Timeout wenigstens eines Zeitgebers (entweder im Therapiegerät oder im externen Gerät);
- Erfassen einer Benutzeraktion, beispielsweise einer Eingabe eines Benutzers oder das an sich bekannte Auflegen eines Magnetes in der Nähe des Implantats;
- das Erfassen eines vorgegebenen Gerätestatus und/oder
- die Detektion einer Störung.

Die Datenkommunikationssteuereinheit kann dabei nach fest vorgegebenen Kriterien arbeiten oder programmierbar sein. Vorzugsweise ist die Datenkommunikationssteuereinheit derart ausgebildet, dass
- eine beliebige Anzahl von Datenübertragungsmodi samt zugehöriger Paketkonfiguration festgelegt ist oder festgelegt werden kann;
- die Wahl eines neuen Datenübertragungsmodus entweder durch das Therapiegerät oder durch das externe Gerät oder durch beide erfolgen kann;
- zur Auswahl eines Datenübertragungsmodus:
   - Timeouts, die festgelegt sind oder festgelegt werden können, die den Übergang von einem beliebigen oder bestimmten Datenübertragungsmodus in einen anderen beliebigen oder bestimmten Datenübertragungsmodus festlegen;
   - Benutzeraktionen festgelegt sind oder festgelegt werden können, die den Übergang und einen beliebigen oder einen bestimmten Datenübertragungsmodus in einen anderen Datenübertragungsmodus festlegen;
   - ein oder mehrere Betriebszustände (Gerätestatus) festgelegt sind oder festgelegt werden können, deren Detektion die Datenkommunikationssteuereinheit veranlasst, einen bestimmten vorgegebenen oder vorgebbaren Datenübertragungsmodus auszuwählen;
   - Störungen festgelegt sind oder festgelegt werden können, deren Detektion die Datenkommunikationssteuereinheit veranlasst, einen vorgegebenen oder vorgebbaren Datenübertragungsmodus auszuwählen;
   - festgelegt sind oder festgelegt werden können, dass der Transceiver in Abhängigkeit von einem Datenübertragungsmodus temporär abgeschaltet wird (in diesem Falle wäre die Datenübertragungsrate für eine vorgegebene Zeit Null);
   - festgelegt ist oder festgelegt werden kann, dass eine temporäre Programmierung durch die Datenkommunikationssteuereinheit aktiviert oder deaktiviert wird in Abhängigkeit von einem ausgewählten Datenübertragungsmodus oder Übergängen zwischen zwei Datenübertragungsmodi.

Wie eingangs erwähnt kann das Therapiegerät auch zwei Therapiegerät-Datenkommunikationsschnittstellen aufweisen, die sich wenigstens hinsichtlich des technischen Aufbaus voneinander unterscheiden. Vorzugsweise können sich die erfindungsgemäß auszuwählenden Datenübertragungsmodi auch durch die Auswahl eines oder mehrerer der zwei Therapiegerät-Datenkommunikationsschnittstellen voneinander unterscheiden. Wie eingangs erwähnt ist vorzugsweise eine Datenkommunikationsschnittstelle dazu ausgebildet, eine Datenübertragung mittels eines elektrischen Wechselfeldes durchzuführen, während die andere Therapiegerät-Datenkommunikationsschnittstelle dazu ausgebildet ist, eine Datenübertragung mit Hilfe eines magnetischen Wechselfeldes durchzuführen.

Entsprechend einer bevorzugten Ausführungsvariante ist die Datenkommunikationssteuereinheit des Therapiegeräts dazu ausgebildet, im Rahmen der Auswahl eines Datenübertragungsmodus genau eine der zur Verfügung stehenden Therapiegerät-Datenkommunikationsschnittstellen auszuwählen und die übrige oder die übrigen abzuschalten.

Vorzugsweise kann eine Therapiegerät-Datenkommunikationsschnittstelle mehrere Schnittstellenkomponenten aufweisen, die jeweils unterschiedliche Betriebszustände - beispielsweise An oder Aus - annehmen können, wobei sich die auszuwählenden Datenübertragungsmodi durch einen jeweiligen Betriebszustand einer oder mehrerer der Schnittstellenkomponenten voneinander unterscheiden können.

Vorzugsweise ist die Datenkommunikationsschnittstelle insbesondere dazu ausgebildet, im Falle des Vorhandenseins einer induktiven Telemetrie, d. h. im Falle des Vorhandenseins eines Programmiergerätes, das über ein magnetisches Wechselfeld mit einer entsprechenden Therapiegerät-Datenkommunikationsschnittstelle kommunizieren kann, den wichtigsten Anteil der übertragenen Daten über die induktive Therapiegerät-Datenkommunikationsschnittstelle zu übertragen und eine Datenübertragung über eine zweite, auf Basis eines elektrischen Wechselfeldes arbeitende Therapiegerät-Datenkommunikationsschnittstelle entweder ganz einzustellen oder nur für zusätzliche, weniger kritische Daten zu verwenden. Solche besonders wichtige, über die induktive Therapiegerät-Datenkommunikationsschnittstelle zu übertragende Daten sind insbesondere Programmierdaten, die wichtigsten Daten von im Therapiegerät gewonnen intrakardialen Elektrokardiogramm, die Übertragung eines Notfallprogramms oder das Auslösen eines Notschocks. Die jeweilige die Auswahl eines Datenübertragungsmodus treffende Datenkommunikationssteuereinheit muss nicht notwendigerweise in demjenigen Gerät (Therapiegerät oder externes Gerät) untergebracht sein, welches die Umschaltung eines Datenübertragungsmodus zuerst durchführt. Beispielsweise ist es möglich, dass eine Benutzeraktion am externen Gerät durchgeführt und von einer Notdatenkommunikationssteuereinheit des externen Geräts erfasst wird. Daraufhin kann die Datenkommunikationsschnittstelle des externen Geräts dazu ausgebildet sein, eine entsprechende Steuersequenz an eine Datenkommunikationssteuereinheit des Therapiegerätes zu übertragen, welches daraufhin den Datenübertragungsmodus wechselt. Eine oder beide an einer jeweiligen Übertragungsstrecke beteiligten Datenkommunikationssteuereinheiten können im Übrigen dazu ausgebildet sein, Indikatordaten zu erzeugen und über die jeweilige Datenübertragungsstrecke zu übertragen, die kennzeichnet, ob ein nächster Datenframe über eine induktive oder eine Hochfrequenzdatenübertragungsstrecke übertragen wird.

Die Erfindung soll nun anhand eines Ausführungsbeispiels mit Bezug auf die Figuren näher erläutert werden. Diese zeigen in:
- Fig. 1:: Eine Übersicht über ein elektrisches Therapiesystem;
- Fig. 2:: eine schematische Blockdarstellung der Komponenten des Therapiesystems aus Fig. 1;
- Fig. 3:: ein Flussdiagramm für die Einleitung der Kommunikation eines Therapiegeräts mit einem externen Gerät;
- Fig. 4:: ein Flussdiagramm welches die Wechsel der Datenübertragungsmodi des Therapiegerätes illustriert (Fortsetzung des Flussdiagramms aus Figur 3).
- Fig. 5: ein Flussdiagramm, welches das Wechseln zwischen verschiedenen Zuständen des Programmiergerätes illustriert.
- Fig. 6: zeigt den nahtlosen Übergang von der induktiven Kommunikation zurück zur HF-Kommunikation.

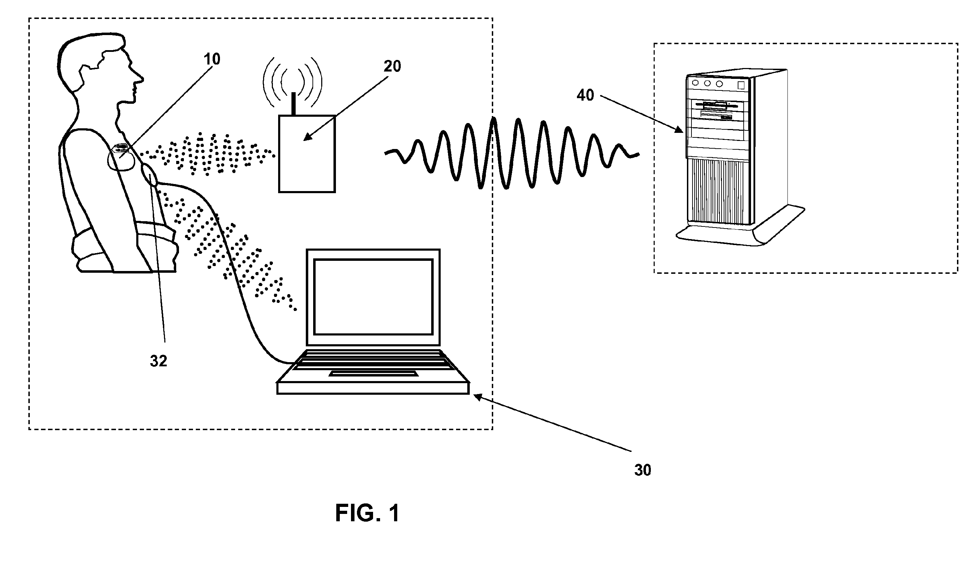

In der Übersicht über ein erfindungsgemäßes Therapiesystem gemäß Fig. 1 ist ein Therapiegerät 10 in Form eines implantierbaren Herzschrittmachers zu erkennen sowie ein Patientengerät 20 als erstes externes Gerät und ein Programmiergerät 30 als zweites externes Gerät. Das Programmiergerät 30 besitzt einen Programmierkopf 32, um eine kurzreichweitige induktive Telemetrieverwendung zum Therapiegerät 10 herstellen zu können, bei der Daten mittels eines magnetischen Wechselfeldes drahtlos zwischen dem Therapiegerät 10 und dem Programmierkopf 32 des Programmiergeräts 30 übertragen werden. Zu diesem Zweck besitzen sowohl das Therapiegerät 10 als auch das Programmiergerät 30 jeweils eine Datenkommunikationsschnittstelle für die induktive Datenübertragung.

Das Therapiegerät 10 besitzt darüber hinaus eine zweite Datenkommunikationsschnittstelle für eine Datenübertragung mittels hochfrequenter elektrischer Wechselfelder, um auf diese Weise drahtlos Daten zwischen dem Therapiegerät 10 und dem Patientengerät 20 oder auch dem Programmiergerät 30 übertragen zu können. Das Patientengerät 20 und das Programmiergerät 30 besitzen jeweils eine entsprechende Datenübertragungsschnittstelle. Das Patientengerät 20 dient im Übrigen als Relaisstation zur Übertragung von Daten zwischen dem Therapiegerät 10 und einem entfernten zentralen Servicecenter 40.

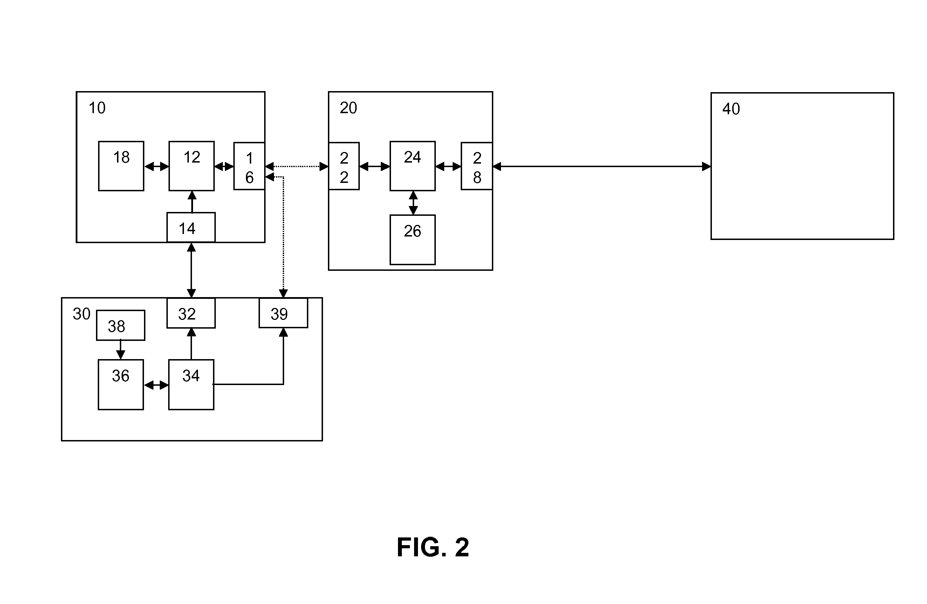

Fig. 2 zeigt die einzelnen Komponenten des Therapiesystems aus Fig. 1 in einer schematischen Blockdarstellung. Dabei sind die Komponenten eines jeweiligen Gerätes des Therapiesystems nur auszugsweise schematisch dargestellt, sofern sie für die vorliegende Erfindung relevant sind. Typische Bestandteile eines Therapiegeräts in Form eines implantierbaren Herzschrittmachers, wie Anschlüsse für Elektrodenleitungen, ein Stimulationspulsgenerator, Sensingeinheiten oder eine Therapiesteuereinheit, sind beispielsweise nicht abgebildet.

Wie Fig. 2 zu entnehmen ist, besitzt das Therapiegerät 10 eine Datenkommunikationssteuereinheit 12, die mit einer ersten Therapiegerät-Datenübertragungsschnittstelle 14 und einer zweiten Therapiegerät-Datenkommunikationsschnittstelle 16 verbunden ist. Die erste Therapiegerät-Datenkommunikationsschnittstelle 14 ist dabei als induktive Datenkommunikationsschnittstelle für die bidirektionale Übertragung von Daten mittels magnetischer Wechselfelder ausgebildet. Die zweite Therapiegerät-Datenkommunikationsschnittstelle 16 ist als Hochfrequenz-Datenkommunikationsschnittstelle zur bidirektionalen Übertragung von Daten mittels hochfrequenter elektrischer Wechselfelder ausgebildet.

Die erste Therapiegerät-Datenkommunikationsschnittstelle 14 dient dabei der Kommunikation mit dem Programmiergerät 30, während die zweite Therapiegerät-Datenkommunikationsschnittstelle 16 einer bidirektionalen Datenkommunikation zwischen dem Therapiegerät 10 und einem Patientengerät 20 oder dem Programmiergerät 30 dient.

Außerdem weist das Therapiegerät 10 eine Therapiegeräte-Steuereinheit 18 auf, die u. a. dazu ausgebildet ist, Betriebszustände des Therapiegeräts 10 zu steuern. Zur Übermittlung von Daten, die wenigstens einige erfasste Betriebszustände kennzeichnen, ist die Therapiegeräte-Steuereinheit 18 mit der Therapiegerät-Datenkommunikationssteuereinheit 12 verbunden.

Das Patientengerät 20 besitzt eine (erste externe) Datenkommunikationsschnittstelle 22, die ausgebildet ist, einem bidirektionalen drahtlosen Datenaustausch mittels eines hochfrequenten elektrischen Wechselfeldes mit der zweiten Therapiegerät-Datenkommunikationsschnittstelle 16 durchzuführen. Die Datenkommunikationsschnittstelle 22 des externen Geräts 20 ist mit einer Datenkommunikationssteuereinheit 24 verbunden, die beispielsweise die Datenkommunikationseinheit 22 hinsichtlich der Parameter des einzusetzenden Protokolls für eine paketorientierte, bidirektionale Datenübertragung steuert. Außerdem ist die Datenkommunikationssteuereinheit 24 ausgebildet, auf Benutzereingabe oder Benutzeraktionen zu reagieren, die ein Benutzer über eine entsprechende Benutzerschnittstelle 26 des Patientengerätes 20 vornehmen kann. Die Benutzerschnittstelle 26 ist dazu mit der Datenkommunikationssteuereinheit 24 des Patientengerätes verbunden.

Außerdem besitzt das Patientengerät 20 noch eine zweite externe Datenkommunikationsschnittstelle 28 zur bidirektionalen Fernübertragung von Daten von zu dem zentralen Servicecenter 40. Diese Datenübertragung kann sowohl drahtlos, beispielsweise über ein Mobilfunknetz oder drahtgebunden stattfinden.

Das Programmiergerät 30 besitzt eine Programmiergerät-Datenkommunikationsschnittstelle 32, die ausgebildet ist, eine bidirektionale Datenübertragung mittels eines magnetischen Wechselfeldes per induktiver Telemetrie mit der induktiven Therapiegerät-Datenkommunikationsschnittstelle 14 durchzuführen. Auch die Programmiergerät-Datenkommunikationsschnittstelle 32 ist mit einer entsprechenden Programmiergerät-Datenkommunikationssteuereinheit 34 verbunden, die dem jeweils zu verwendenden Datenübertragungsmodus, insbesondere das Protokoll zur paketorientierten und bidirektionalen Datenübertragung steuert. Die Programmiergerät-Datenkommunikationssteuereinheit 34 ist mit einer zentralen Programmiergerätsteuereinheit 36 verbunden, die ihrerseits wiederum mit einer Benutzerschnittstelle 38 verbunden ist, die es erlaubt, Benutzereingaben zur Steuerung des Programmiergerätes 30 und insbesondere auch zur Steuerung der Datenübertragung zwischen dem Programmiergerät 30 und dem Therapiegerät 10 entgegenzunehmen.

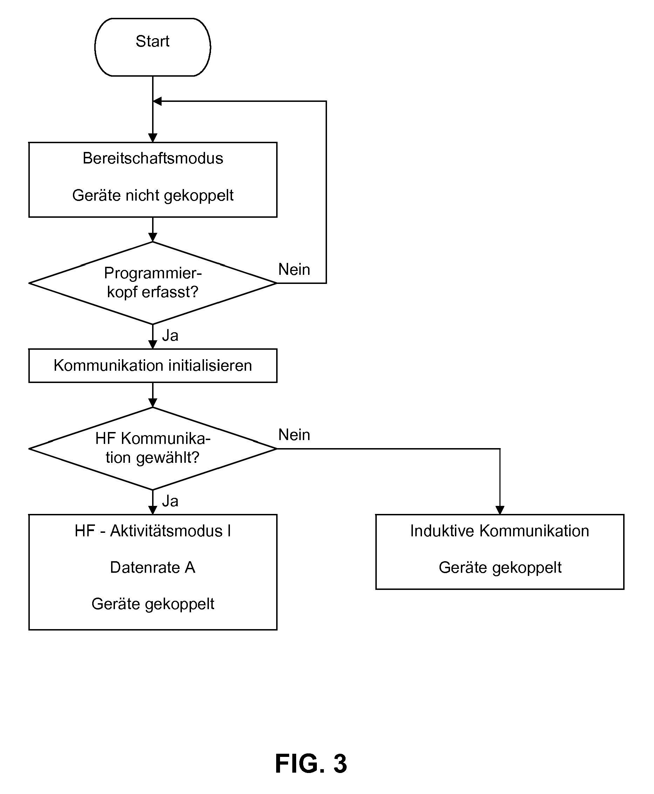

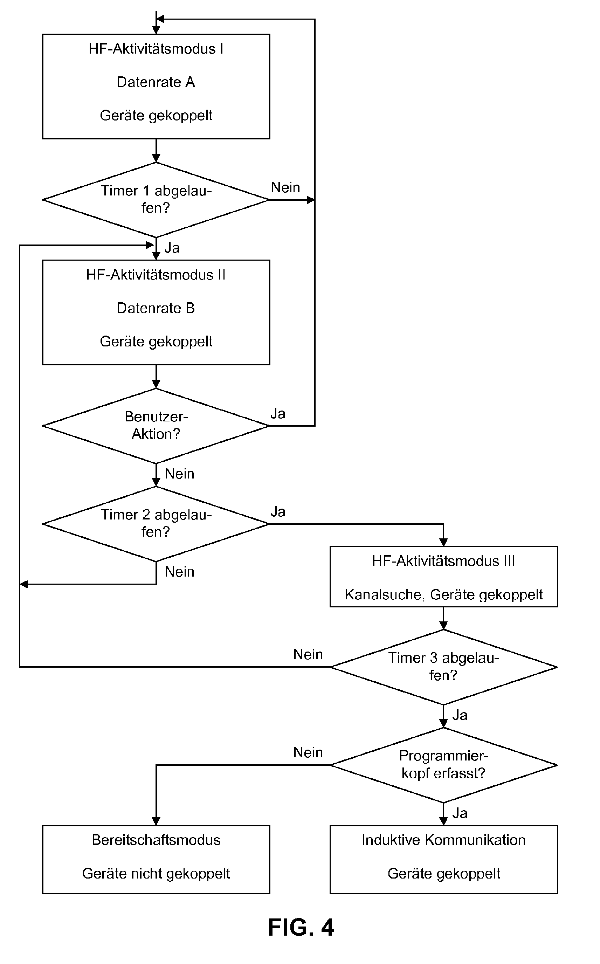

In Bezug auf eine induktive Telemetrie zwischen dem Therapiegerät 10 und dem Programmiergerät 30 ist die erste, induktive Therapiegerät-Datenkommunikationsschnittstelle 14 dazu ausgebildet, die Annäherung eines Programmmierkopfes des Programmiergerätes 30 der die Programmiergerät-Datenkommunikationsschnittstelle 32 beinhaltet, selbsttätig zu erfassen. Dieses Erfassen der Annäherung eines Programmierkopfes 32 ist gemäß einer bevorzugten Ausführungsvariante der Erfindung ein Ereignis, welches die Therapiegerät-Datenkommunikationssteuereinheit 12 bei der Auswahl eines jeweiligen anzuwendenden Datenübertragungsmodus berücksichtigt. Weitere Ereignisse und Regeln, die das Verhalten der Therapiegerät-Datenkommunikationssteuereinheit 12 aufgrund entsprechender Implementierung dieser Datenkommunikationssteuereinheit beeinflussen und steuern, werden nun nachfolgend anhand der Zustandsdiagramme in den Figuren 3 und 4 näher erläutert.

Wie Figur 2 zu entnehmen ist und in Figur 1 bereits angedeutet ist, besitzt in einer bevorzugten Ausführungsvariante auch das Programmiergerät 30 eine zweite Programmiergerät-Datenkommunikationsschnittstelle 39, die zur bidirektionalen Datenübertragung zwischen der zweiten Programmiergerät-Datenkommunikationsschnittstelle 39 und der zweiten Therapiegerät-Datenkommunikationsschnittstelle 16 über ein hochfrequentes elektrisches Wechselfeld ausgebildet ist. Die Programmiergerät-Datenkommunikationssteuereinheit 34 kann im Rahmen der Auswahl eines Datenübertragungsmodus die erste Programmiergerät-Datenkommunikationsschnittstelle 32, die zweite Programmiergerät-Datenkommunikationsschnittstelle 39 oder beide auswählen.

Fig. 3 bis 5 erläutern die Zustandsübergänge zwischen den einzelnen HF Datenübertragungsmodi. In den Figuren sind diese jeweils als HF Aktivitätsmodus bezeichnet. Sie führen zum Ende entweder in den Bereitschaftsmodus oder in die Induktive Kommunikation. Aus Gründen der Übersichtlichkeit ist im Flussdiagramm nicht dargestellt, dass im HF-Aktivitätsmodus 1 oder 11 die induktive Kommunikation durch Auflegen des Programmierkopfes erzwungen werden kann. Dies ist mittels einer zusätzlichen Abfrage, ob nach Empfang eines Datenpaketes der Programmierkopf erkannt wurde, möglich. Diese Abfrage und die daran anknüpfenden Schritte hätten die Zeichnung zu unübersichtlich gemacht.

Figuren 3 und 4 betreffen die Datenübertragungsmodi des Therapiegerätes 10 für die Datenübertragung über die zweite Therapiegerät-Datenkommunikationsschnittstelle mittels eines hochfrequenten elektrischen Wechselfeldes. Diese Datenübertragungsmodi I bis III sind in Figuren 3 und 4 als HF-Aktivitätsmodi I bis III bezeichnet. Ein vierter Datenübertragungsmodus ist die induktive Kommunikation zwischen Therapiegerät und Programmiergarät. Somit sind für das Therapiegerät 10 insgesamt vier Datenübertragungsmodi vorgesehen, nämlich drei HF-Aktivitätsmodi I bis III und ein induktiver Komunikationsmodus. Das Wechseln zwischen einzelnen Datenübertragungsmodi des Therapiegerätes 10 ist durch dessen Therapiegerät-Datenkommunikationssteuereinheit 12 gesteuert.

Fig. 3 zeigt konkret, wie das Therapiegerät 10 eine Datenübertragung mit einem Programmiergerät 30 aufbaut. Ursprünglich befindet sich das Therapiegerät in einem Betriebsmodus, in dem das Therapiegerät mit keinem externen Gerät, also auch insbesondere mit keinem Programmiergerät 30, gekoppelt ist. Dieser Betriebsmodus wird als Bereitschaftsmodus bezeichnet. In diesem Betriebsmodus fragt das Therapiegerät 10 regelmäßig ab, ob es über seine induktive Datenkommunikationsschnittstelle 14 die Annäherung eines Programmierkopfs erfasst. Ist dies nicht der Fall, verbleibt das Therapiegerät 10 im Bereitschaftsmodus. Für den Fall, dass die induktive Datenkommunikationsschnittstelle 14 einen Programmierkopf erfasst, wird eine Datenkommunikation eingeleitet (Datenkommunikation initialisieren). Im Rahmen eines Datenaustausches während des Einleitens der Datenkommunikation prüft das Therapiegerät, ob die übertragenen und empfangenen Daten indizieren, dass seitens des Programmiergeräts 30 eine Kommunikation mittels eines hochfrequenten elektrischen Wechselfeldes gewünscht ist (HF Kommunikation gewählt?). Falls dies der Fall ist, aktiviert das Therapiegerät 10 die zweite Datenkommunikationsschnittstelle 16 (Hochfrequenzdatenkommunikationsschnittstelle 16) für die bidirektionale Übertragung von Daten. Für diesen Fall ist das Therapiegerät 10 ausgebildet, einen ersten Datenübertragungsmodus I (in Fig. 3 als HF-Aktivitätsmodus I bezeichnet) anzunehmen, in dem Daten mit einer möglichst hohen Datenrate A zwischen dem Therapiegerät 10 und dem Programmiergerät 30 übertragen werden können. Das Therapiegerät 10 befindet sich dann in einem Zustand, in dem das Therapiegerät 10 mit dem Programmiergerät 30 gekoppelt ist.

Falls die seitens des Programmiergerätes 30 während der Einleitung der Datenkommunikation empfangenen Daten indizieren, dass die Kommunikation über ein hochfrequentes elektrisches Wechselfeld nicht gewünscht ist, initialisiert das Therapiegerät 10 einen induktiven Datenübertragungsmodus (induktive Kommunikation), in dem das Therapiegerät 10 und das Programmiergerät 30 für eine drahtlose Kommunikation über die jeweilige induktive Datenkommunikationsschnittstelle eingerichtet sind. Die Therapiegerät-Datenkommunikationssteuereinheit 12 ist somit so ausgebildet, dass sie auf ein Einschalten hin zunächst den Datenübertragungsmodus I (HF-Aktivitätsmodus I) aktiviert, indem eine Datenübertragung mit einem Protokoll für eine paketorientierte Datenübertragung durchgeführt wird, welches die höchstmögliche Datenrate A erlaubt.

Unter Umständen geht das Therapiegerät 10 selbständig in einen anderen Datenübertragungsmodus über, der beispielsweise mit einer niedrigeren Datenrate verbunden ist. Dies ist in Fig. 4 illustriert. Zunächst befindet sich das Therapiegerät 10 in dem Datenübertragungsmodus I mit höchster Datenübertragungsrate. In diesem Datenübertragungsmodus I nach Initiierung der Datenübertragung sind das Therapiegerät 10 und das zugehörige Programmiergerät 30 gekoppelt und die bidirektionale Datenübertragung findet zwischen diesen beiden Geräten statt. Während einer solchen aktiven Datenverbindung reagiert die Datenkommunikationssteuereinheit 12 auf Eingaben eines Benutzers. Außerdem läuft während einer aktiven Datenverbindung ständig ein erster Zeitgeber Timer 1 ab, der zurückgesetzt wird, wenn entweder eine Benutzereingabe erfolgt oder die erforderliche Datenrate höher ist als die Datenrate B im Datenübertragungsmodus II. Wenn keine Benutzereingabe erfolgt beziehungsweise die erforderliche Datenrate ständig kleiner ist als die Datenrate B, läuft der erste Zeitgeber (Timer 1) über fünf Minuten und endet daraufhin mit einem Timeout. In diesem Falle initiiert die Therapiegerät-Datenkommunikationssteuereinheit 12 einen Wechsel vom Datenübertragungsmodus l in einen Datenübertragungsmodus II (HF-Aktivitätsmodus II), indem die Datenverbindung zwischen dem Therapiegerät 10 und dem Programmiergerät 30 nach wie vor aktiv ist, jedoch ein Protokoll für die paketorientierte bidirektionale Datenübertragung verwendet wird, welches eine andere Paket-Konfiguration aufweist, die eine niedrigere Datenrate B zur Folge hat und weniger Energie benötigt, als die Datenübertragung im Datenübertragungsmodus I mit der Datenrate A.

Erfolgt während des HF-Aktivitätsmodus II eine Benutzereingabe (Benutzeraktion) so initiiert die Therapiegerät-Datenkommunikationssteuereinheit 12 einen Wechsel vom Datenübertragungsmodus II zurück in den Datenübertragungsmodus I. Während sich das Therapiegerät 10 in diesem Datenübertragungsmodus II befindet, ist ein zweiter Zeitgeber (Timer 2) aktiv, der ein Timeout nach einer Sekunde hat und immer dann zurückgesetzt wird, wenn die Datenkommunikationsschnittstelle 16 des Therapiegeräts ein Datenpaket empfängt. Empfängt die zweite Datenkommunikationsschnittstelle 16 des Therapiegeräts 10 eine Sekunde lang kein Datenpaket, kommt es zu einem Timeout des zweiten Zeitgebers mit der Folge, dass die Datenkommunikationssteuereinheit 12 des Therapiegeräts 10 einen Wechsel vom Datenübertragungsmodus II in einen Datenübertragungsmodus III (HF-Aktivitätsmodus III), in dem das Therapiegerät 10 und das zugehörige Programmiergerät 30 weiter miteinander gekoppelt sind. In diesem Datenübertragungsmodus III führt die zweite Datenkommunikationsschnittstelle 16 des Therapiegerätes 10 eine Kanalsuche nach evtl. aktiven (weiteren) Datenübertragungskanälen durch. Gleichzeitig wird ein dritter Zeitgeber (Timer 3) gestartet, der ein Timeout nach fünf Minuten hat und dann zurückgesetzt wird, wenn die Kanalsuche im Datenübertragungsmodus III dazu führt, dass die zweite Datenkommunikationsschnittstelle 16 einen aktiven Datenübertragungskanal findet. Dann kehrt das Therapiegerät 10 in den Datenübertragungsmodus II zurück und benutzt hierfür den gefundenen aktiven Datenübertragungskanal.

Kommt es zu einem Timeout des dritten Zeitgebers (Timer 3) nach fünf Minuten, fragt das Therapiegerät 10 die erste induktive Datenkommunikationsschnittstelle 14 ab und prüft, ob diese erste Datenkommunikationsschnittstelle 14 die Annäherung eines Programmierkopfes erfasst. Ist dies der Fall, geht das Therapiegerät 10 in den induktiven Datenübertragungsmodus über, in dem das Therapiegerät 10 und das Programmiergerät 30 über die beiden induktiven Datenkommunikationsschnittstellen miteinander gekoppelt sind.

Ist der dritte Zeitgeber nach fünf Minuten abgelaufen und wird danach über die erste Datenkommunikationsschnittstelle 14 keine Annäherung eines Programmierkopfes erfasst, geht das Therapiegerät 10 wieder in den Bereitschaftsmodus über, in dem es nicht mit einem Programmiergerät 30 gekoppelt ist. Die zweite Datenkommunikationsschnittstelle 16 wird dann abgeschaltet und die Datenverbindung abgebrochen. Das Therapiegerät 10 und das Programmiergerät 30 sind nicht mehr miteinander gekoppelt.

Weitere Details können dem Zustandsdiagramm in Figuren 3 und 4 entnommen werden. Aus Gründen der Übersichtlichkeit ist im Flussdiagramm nicht dargestellt, dass im Datenübertragungsmodus I oder II jederzeit durch Auflegen des Programmierkopfes ein Übergang in den induktiven Datenkommunikationsmodus erzwungen werden kann.

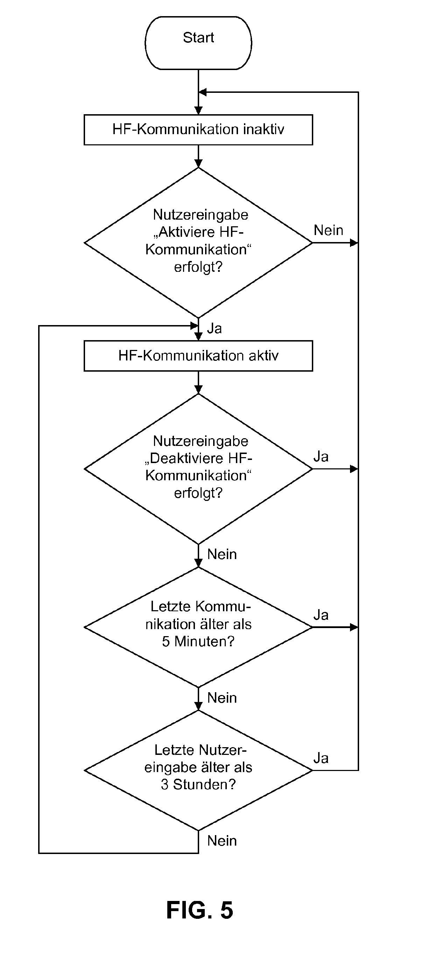

Das Flussdiagramm in Fig. 5 zeigt die möglichen Datenübertragungsmodi des Programmiergeräts 30 in Bezug auf eine Datenübertragung mittels der zweiten Programmiergerät-Datenkommunikationsschnittstelle 39 über ein hochfrequentes elektrisches Wechselfeld. Wie Fig. 5, zu entnehmen ist, kennt das Programmiergerät 30 zwei Datenübertragungsmodi, nämlich einen ersten Datenübertragungsmodus, bei dem die Datenübertragung über ein hochfrequentes elektrisches Wechselfeld aktiv ist und einen zweiten Datenübertragungsmodus, bei dem die Datenübertragung über ein hochfrequentes elektrisches Wechselfeld inaktiv ist. Ein Wechsel zwischen diesen beiden Datenübertragungsmodi erfolgt entweder durch gezieltes Ein- und Ausschalten, also ein über die Benutzerschnittstelle 38 einzugebendes und von der Programmiergerät-Datenkommunikationsschnittstelle 34 zu verarbeitendes Ereignis. Ein Übergang vom aktiven Datenübertragungsmodus zum inaktiven Datenübertragungsmodus des Programmiergerätes 30 kann jedoch auch Zeitgeber-gesteuert erfolgen. Demnach erfolgt der Übergang vom aktiven Datenübertragungsmodus zum inaktiven Datenübertragungsmodus entweder nach Timeout eines ersten Programmiergerät-Zeitgebers wenn die Programmiergerät-Datenkommunikationssteuereinheit 34 für mehr als fünf Minuten nach Aussenden des letzten Datenpaketes kein Datenpaket seitens des Therapiegerät 10 empfängt, oder nach Timeout eines zweiten Programmiergerät-Zeitgebers, wenn die Steuereinheit 36 des Programmiergeräts 30 drei Stunden lang keine Benutzereingabe erfasst.

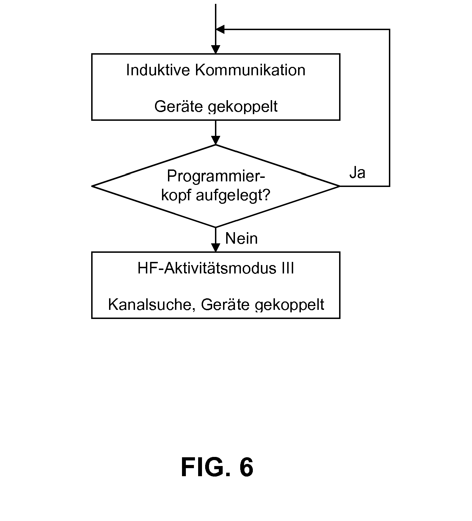

Figur 6 illustriert schließlich, dass ein Wechsel zwischen einer Datenübertragung mittels hochfrequenten elektrischen Wechselfeldes und induktiver Datenübertragung nahtlos erfolgt. Sobald der Programmierkopf entfernt wurde, kehrt das Therapiegerät 10 automatisch in den Datenübertragungsmodus III (HF-Aktivitätsmodus III) zurück.

Zusammengefasst ergibt sich ein Therapiesystem und ein Therapiegerät mit wenigstens einer Datenkommunikationsschnittstelle, die in verschiednen Datenübertragungsmodi arbeiten kann und mit einer Datenkommunikationssteuereinheit derart zusammenwirkt, dass die Datenkommunikationsschnittstelle gesteuert von der Datenkommunikationssteuereinheit in Abhängigkeit vorgegebener Auswahlkriterien ohne Unterbrechung einer bestehenden Datenverbindung von einem in einen anderen Datenübertragungsmodus wechseln kann.

## Patentansprüche

1. Elektrisches Therapiesystem mit einem Therapiegerät (10) und einem Programmiergerät (30), von denen das Therapiegerät (10) zwei Therapiegerät-Datenkommunikationsschnittstellen (14; 16) aufweist, von denen die erste Thera-piegerät-Datenübertragungsschnittstelle (14) als induktive Datenkommunikationsschnittstelle für die bidirektionale Übertragung von Daten mittels magnetischer Wechselfelder ausgebildet ist und die zweite Therapiegerät-Datenkommunikationsschnittstelle (16) als Hochfrequenz-Datenkommunikationsschnittstelle zur bidirektionalen Übertragung von Daten mittels hochfrequenter elektrischer Wechselfelder ausgebildet ist, und von denen das Programmiergerät (30) zwei Programmiergerät-Datenkommunikationsschnittstellen (32; 39) aufweist, von denen die erste Programmiergerät-Datenkommunikationsschnittstelle (32) ausgebildet ist, eine bidirektionale Datenübertragung mittels eines magnetischen Wechselfeldes mit der induktiven Therapiegerät-Datenkommunikationsschnittstelle (14) des Therapiegerätes (10) durchzuführen und von denen die zweite Programmiergerät-Datenkommunikationsschnittstelle (39) ausgebildet ist, eine bidirektionale Datenübertragung mit der zweiten Therapiegerät-Datenkommunikationsschnittstelle (16) des Therapiegerätes (10) über ein hochfrequentes elektrisches Wechselfeld durchzuführen,
wobei sowohl die Therapiegerät-Datenkommunikationsschnittstellen (14; 16) als auch die Programmiergerät-Datenkommunikationsschnittstellen (32; 39) mit jeweils einer Datenkommunikationssteuereinheit (12; 34) verbunden sind, die ausgebildet ist, die jeweiligen Datenkommunikationsschnittstellen zu steuern,
wobei eine jeweilige Datenübertragung in einem von wenigstens zwei Datenübertragungsmodi erfolgt,
**dadurch gekennzeichnet dass** wenigstens eine der beiden Datenkommunikationssteuereinheiten (12; 34) ausgebildet ist, eine oder beide Datenkommunikationsschnittstellen mit einem jeweils anzuwendenden Datenübertragungsmodus anhand wenigstens eines vorgegebenen Auswahlkriteriums auszuwählen und beim Auftreten eines einem Auswahlkriterium entsprechenden Ereignisses derart von einem Datenübertragungsmodus in einen anderen zu wechseln, dass eine bestehende Datenverbindung eines Datenübertragungsmodus nicht
unterbrochen wird, und die Datenübertragung über die Datenverbindung eines anderen Datenübertragungsmodus fortgesetzt wird.

2. Elektrisches Therapiesystem nach Anspruch 1, **dadurch gekennzeichnet, dass** sich die Datenübertragungsmodi wenigstens hinsichtlich der Paketkonfiguration und/oder des Protokolls für die Datenübertragung voneinander unterscheiden.

3. Elektrisches Therapiesystem nach Anspruch 1 oder 2, **dadurch gekennzeichnet, dass** das wenigstens eine vorgegebene Auswahlkriterium ein Auswahlkriterium aus der folgenden Liste ist:
- Timeout wenigstens eines Zeitgebers,
- Benutzeraktion,
- Gerätestatus und/oder
- Detektion einer Störung.

4. Elektrisches Therapiesystem nach Anspruch 1, **dadurch gekennzeichnet, dass** die Datenkommunikationssteuereinheit (12) des Therapiegerätes (10) ausgebildet ist, im Rahmen der Auswahl eines Datenübertragungsmodus einer der beiden Therapiegerät-Datenkommunikationsschnittstellen (14, 16) auszuwählen und die jeweils andere abzuschalten.

5. Elektrisches Therapiesystem nach einem der Ansprüche 1 bis 4, **dadurch gekennzeichnet, dass** wenigstens eine Therapiegerät-Datenkommunikationsschnittstelle (14, 16) mehrere Schnittstellenkomponenten aufweist, die jeweils unterschiedliche Betriebszustände annehmen können und sich die Datenübertragungsmodi durch einen jeweiligen Betriebszustand einer Schnittstellenkomponente voneinander unterscheiden können.

6. Elektrisches Therapiesystem nach Anspruch 5, **dadurch gekennzeichnet, dass** die Betriebszustände wenigstens einen eingeschalten Zustand und einen ausgeschalten Zustand umfassen.

7. Therapiegerät für ein elektrisches Therapiesystem nach einem der Ansprüche 1 bis 6, **dadurch gekennzeichnet, dass** die Therapiegerät-Datenkommunikationssteuereinheit (12) ausgebildet ist, auf über die Therapiegerät-Datenkommunikationsschnittstelle empfangenen Steuersignale anzusprechen und einen jeweils anzuwendenden Datenübertragungsmodus in Abhängigkeit eines empfangenen Steuersignals auszuwählen.

8. Therapiegerät nach Anspruch 7, **dadurch gekennzeichnet, dass** das Therapiegerät (10) ausgebildet ist, die Nähe eines Programmierkopfes für eine induktive Datenübertragung mittels eines magnetischen Wechselfeldes zu erfassen und die Therapiegerät-Datenkommunikationssteuereinheit (12) ausgebildet ist, einen Datenübertragungsmodus in Abhängigkeit davon zu auszuwählen, ob sich ein Programmierkopf in der Nähe des Therapiegerätes befindet oder nicht.

## Claims

1. An electric therapy system comprising a therapy device (10) and a programming device (30), of which the therapy device (10) has two therapy device data communication interfaces (14; 16), of which the first therapy device data transmission interface (14) is designed as an inductive data communication interface for the bidirectional transmission of data by means of magnetic alternating fields and the second therapy device data communication interface (16) is designed as a high frequency data communication interface for the bidirectional transmission of data by means of high-frequency electric alternating fields, and of which the programming device (30) has two programming device data communication interfaces (32; 39), of which the first programming device data communication interface (32) is designed to carry out a bidirectional data transmission by means of a magnetic alternating field with the inductive therapy device data communication interface (14) of the therapy device (10), and of which the second programming device data communication interface (39) is desgiend to carry out a bidirectional data transmission with the second therapy device data communication interface (16) of the therapy device (10) via a high-frequency, electric alternating field, wherein the therapy device data communication interfaces (14; 16) as well as the programming device data communication interfaces (32; 39) are each connected to a respective data communication control unit (12; 34), which is designed to control the respective data communication interfaces,
wherein a respective data transmission takes place in one of at least two data transmission modes,
**characterized in that** at least one of the two data communication control units (12; 34) is designed to select, on the basis of at least one predetermined selection criterium, one or both data communication interfaces with a respective data transmission mode to be used and, if an event occurs that corresponds to a selection criterium, is designed to switch from one data transmission mode to another such that an existing data connection of a data transmission mode is not interrupted, and the data transmission is continued via the data connection of another data transmission mode.

2. The electric therapy system according to claim 1, **characterized in that** the data transmission modes differ from one another at least in terms of the packet configuration and/or the protocol for the data transmission.

3. The electric therapy system according to claim 1 or 2, **characterized in that** the at least one predetermined selection criterium is a selection criterium from the following list:
- timeout of at least one timer,
- user action,
- device status and/or
- detection of an interference.

4. The electric therapy system according to claim 1, **characterized in that** the data communication control unit (12) of the therapy device (10) is designed such that, within the scope of the selection of a data transmission mode, one of the two therapy device data communication interfaces (14, 16) is selected and the other one is switched off.

5. The electric therapy system according to any one of claims 1 to 4, **characterized in that** at least one therapy device data communication interface (14, 16) has a plurality of interface components, each of which can assume different operating states, and the data transmission modes can differ from one another by a respective operating state of an interface component.

6. The electric therapy system according to claim 5, **characterized in that** the operating states comprise at least one switched-on state and one switched-off state.

7. A therapy device for an electric therapy system according to any one of claims 1 to 6, **characterized in that** the therapy device data communication control unit (12) is designed to respond to control signals received via the therapy device data communication interface and to select a respective data transmission mode to use depending on a control signal that was received.

8. The therapy device according to claim 7, **characterized in that** the therapy device (10) is designed to detect the proximity of a programming head for an inductive data transmission by means of a magnetic alternating field, and the therapy device data communication control unit (12) is designed to select a data transmission mode depending on whether or not a programming head is located in the proximity of the therapy device.

## Revendications

1. Système de thérapie électrique avec un appareil de thérapie (10) et un appareil de programmation (30), parmi lesquels l'appareil de thérapie (10) comporte deux interfaces de communication de données d'appareil de thérapie (14 ; 16), parmi lesquelles la première interface de communication de données d'appareil de thérapie (14) est conçue sous la forme d'une interface de communication de données inductive pour la transmission bidirectionnelle de données au moyen de champs alternatifs magnétiques et la deuxième interface de communication de données d'appareil de thérapie (16) est conçue sous la forme d'une interface de communication de données haute fréquence pour la transmission bidirectionnelle de données au moyen de champs alternatifs électriques de haute fréquence, et parmi lesquels l'appareil de programmation (30) comporte deux interfaces de communication de données d'appareillage de programmation (32 ; 39), parmi lesquelles la première interface de communication de données d'appareillage de programmation (32) est conçue pour effectuer la transmission bidirectionnelle de données au moyen d'un champ alternatif magnétique avec l'interface de communication de données d'appareil de thérapie (14) inductif de l'appareil de thérapie (10), et parmi lesquelles la deuxième interface de communication de données d'appareillage de programmation (39) est conçue pour effectuer une transmission bidirectionnelle de données avec la deuxième interface de communication de données d'appareil de thérapie (16) de l'appareil de thérapie (10) par l'intermédiaire d'un champ alternatif électrique de haute fréquence,
où, à la fois, les interfaces de communication de données d'appareil de thérapie (14 ; 16) et également les interfaces de communication de données d'appareillage de programmation (32 ; 39) sont reliées respectivement avec une unité de commande de communication de données (12 ; 34), qui est conçue pour commander les interfaces de communication de données respectives,
où une transmission de données respectif est effectué selon un mode parmi au moins deux modes de transmission de données,
**caractérisé en ce qu'**au moins l'une des deux unités de commande de communication de données (12 ; 34) est conçue pour choisir une ou deux interfaces de communication de données avec un mode de transmission de données à employer respectivement en tenant compte d'au moins un critère de sélection prédéfini et, dans le cas où des événements correspondant à un critère de sélection se produisaient, passer dans un autre mode de transmission de données, de sorte que la liaison des données en place par un mode de transmission de données n'est pas interrompue, et que la transmission de données est poursuivi par l'intermédiaire d'une liaison de données dans un autre mode de transmission de données.

2. Système de thérapie électrique selon la revendication 1, **caractérisé en ce que** les modes de transmission de données se différentient l'un de l'autre au moins en ce qui concerne la configuration des boîtiers et/ou le protocole pour la transmission de données.

3. Système de thérapie électrique selon les revendications 1 ou 2, **caractérisé en ce qu'**au moins un critère de sélection prédéfini est un critère de sélection faisant partie de la liste suivante :
- temps mort d'au moins un indicateur de temps,
- action de l'utilisateur,
- état de l'appareil et/ou
- détection d'une anomalie.

4. Système de thérapie électrique selon la revendication 1, **caractérisé en ce que** l'unité de commande de communication de données (12) de l'appareil de thérapie (10) est conçue pour choisir l'une des deux interfaces de communication de données d'appareil de thérapie (14, 16) dans le cadre du choix d'un mode de transmission de données et de couper respectivement l'autre.

5. Système de thérapie électrique selon l'une des revendications 1 à 4, **caractérisé en ce qu'**au moins une interface de communication de données d'appareil de thérapie (14, 16) présente plusieurs composantes d'interface, qui peuvent admettre chacune des états de fonctionnement différents et que les modes de transmission de données peuvent se différentier l'un de l'autre par un état de fonctionnement respectif d'une composante d'interface.

6. Système de thérapie électrique selon la revendication 5, **caractérisé en ce que** les états de fonctionnement comprennent au moins un état branché et un état débranché.

7. Appareil de thérapie pour un système de thérapie électrique selon l'une des revendications 1 à 6, **caractérisé en ce que** l'unité de commande de communication de données d'appareil de thérapie (12) est conçue pour appréhender les signaux de commande reçus par l'intermédiaire de l'interface de communication de données d'appareil de thérapie et pour choisir respectivement un mode de transmission de données à employer en fonction d'un signal de commande reçu.

8. Appareil de thérapie selon la revendication 7, **caractérisé en ce que** l'appareil de thérapie (10) est conçu pour détecter la proximité d'une tête de programmation pour une transmission de données au moyen d'un champ alternatif magnétique et l'unité de commande de communication de données d'appareil de thérapie (12) est conçue pour choisir un mode de transmission de données en fonction du fait qu'une tête de programmation se trouve ou non à proximité de l'appareil de thérapie.
